# EUROPEAN PATENT SPECIFICATION

(11) **EP 4 457 589 B1**
(45) Date of publication and mention of the grant of the patent: **10.06.2026**
(21) Application number: 21970126.5
(22) Date of filing: 27.12.2021
(51) Int. Cl.: G06F 3/01, G06T 19/00, A61B 5/00, G05B 19/04

(54) **METHODS AND MEANS FOR RENDERING EXTENDED REALITY**
VERFAHREN UND MITTEL ZUR DARSTELLUNG ERWEITERTER REALITÄT
PROCÉDÉS ET MOYENS DE RENDU DE RÉALITÉ ÉTENDUE

(43) Date of publication of application: 06.11.2024
(73) Proprietor: Telefonaktiebolaget LM Ericsson (publ), 164 83 Stockholm (SE)
(72) Inventor: LINDSKOG, Niklas, 224 72 LUND (SE); NORMANN, Henrik, 215 68 Malmö (SE); ÖKVIST, Peter, 973 41 Luleå (SE); ARNGREN, Tommy, 954 42 Södra Sunderbyn (SE)
(74) Representative: Ericsson
(86) International application number: PCT/SE2021/051315
(87) International publication number: WO 2023/128841

(56) References cited:
- WO-A1-2016/164178
- WO-A1-2019/068025
- WO-A1-2021/061449
- US-A1- 2018 190 020
- US-A1- 2019 064 919
- US-A1- 2021 303 070
- US-A1- 2021 400 342
- US-B2- 10 325 409
- SAMSUNG ELECTRONICS CO., LTD. (RAPPORTEUR): "[FS_5GSTAR] Permanent Document", 3GPP DRAFT; S4-210875, 3RD GENERATION PARTNERSHIP PROJECT (3GPP), MOBILE COMPETENCE CENTRE ; 650, ROUTE DES LUCIOLES ; F-06921 SOPHIA-ANTIPOLIS CEDEX ; FRANCE, vol. TSG SA, no. Electronics; 20210504, 26 May 2021 (2021-05-26), Mobile Competence Centre ; 650, route des Lucioles ; F-06921 Sophia-Antipolis Cedex ; France , XP052015179

## Description

### TECHNICAL FIELD

The technology disclosed herein relates generally to the field of content filtering of computer-generated content creating an extended reality, and in particular to means and methods for rendering Extended Reality, XR, content to a user.

### BACKGROUND

Extended reality (XR) is a term referring to all real-and-virtual environments and to human - machine interactions generated by computer technology and wearables. Virtual Reality (VR), Augmented Reality (AR) and Mixed Reality (MR) are examples on XRs, where the "X" of XR is a variable indicating any current or future spatial computing technology.

A person may be completely immersed into an XR environment and might in such situations run the risk of getting a cognitive overload, in which the person gets too much information and/or too many tasks simultaneously and becomes unable to process the information. He may then, depending on the physical environment he is in, be prone to injuries or accidents. It may therefore be desirable or even necessary to remove or filter content of digital media (computer graphics, sound etc.) in XR. Such removal or filtering is often cumbersome, and failure thereof results in provision of irrelevant content, which in turn gives a poor user experience. In some use cases, XR glasses are needed in order to present necessary information for the user and in these use cases the risk of the cognitive overload is high, as well as the risk for injuries and accidents.

Classic information filters, such as spam filters, are inadequate in XR environments, which are highly dynamic. The classic information filters are also inadequate when it comes to providing the information of interest for the user at a certain point in time.

The prior art US 10325409 B2 deals with automatically classifying real-world objects and automatically associating an appropriate software application to be executed on a mixed reality device, to consistently overlay physical world elements in the virtual world to improve the experience of a user who is visualizing a scene on the mixed reality device HMD.

There is a need for improving safety and user experience in XR environments.

### SUMMARY

An objective of embodiments herein is to address and improve various aspects for personal safety in XR experience, by preventing, for instance, accidents and cognitive overload from happening to the user during an XR event. This objective and others are achieved by the methods, devices, computer programs and computer program products according to the appended independent claims, and by the embodiments according to the dependent claims.

This objective and others are accomplished by using, inter alia, various policies, and classification of content as basis in order to dynamically determine what information to present to the user.

According to a first aspect there is presented a method for rendering Extended Reality, XR, content to a user. The method is performed in a device and comprises receiving, in the device, XR content; determining, in a policy entity, the XR content to be rendered based on one or more policies; classifying, in a classification service, the XR content; and rendering the XR content in an XR environment based on the one or more policies and the classification of the XR content.

In an aspect related to the above, a current cognitive load of the user is determined and taken into account when determining whether to render particular XR content.

According to a second aspect there is presented a computer program for rendering Extended Reality, XR, content to a user. The computer program comprises computer code which, when run on processing circuitry of a device, causes the device to perform a method according to the first aspect.

According to a third aspect there is presented a computer program product comprising a computer program as above, and a computer readable storage medium on which the computer program is stored.

According to a fourth aspect there is presented a device for providing Extended Reality, XR, content to a user. The device is configured to: receive XR content; determine which XR content to render based on one or more policies; classify the XR content; and render XR content in an XR environment based on the one or more policies and the classification of the XR content.

According to a fifth aspect there is presented a method for rendering Extended Reality, XR, content to a user. The method is performed in a system and comprises receiving XR content; determining the XR content to be rendered based on one or more policies; classifying the XR content; and rendering the XR content in an XR environment based on the one or more policies and the classification of the XR content.

According to a sixth aspect there is presented a system for rendering Extended Reality, XR, content to a user. The system is configured to: receive XR content; determine the XR content to be rendered based on one or more policies; classify the XR content; and render the XR content in an XR environment based on the one or more policies and the classification of the XR content.

Advantageously, these aspects enable an XR experience accounting for, and reducing, risks of personal injuries. By taking into account and using factors such as, for instance, various policies and classifications, cognitive load, proximity to objects and direction of user in the information filtering, the personal safety during XR events is improved. Further, XR content may be altered and/or displaced before rendering it if a policy is updated, or the currently displayed content may be adapted if a corresponding policy is updated. These aspects enable decisions to be made dynamically based on various factors, which factors may change during the XR experience.

Advantageously, these aspects are applicable in various scenarios, and in particular in virtual environments or in a real environment with augmented content presented as an overlay. In such scenarios information is presented in e.g., XR glasses and the risk of cognitive overload is high. This may, for instance, be the case when a user is unable or unsuited to manually interact with the content in the XR environment, e.g., when driving a car or performing tasks wherein both hands are occupied.

Other objectives, features and advantages of the enclosed embodiments will be apparent from the following detailed disclosure, from the attached dependent claims as well as from the drawings.

Generally, all terms used in the claims are to be interpreted according to their ordinary meaning in the technical field, unless explicitly defined otherwise herein. All references to "a/an/the element, apparatus, component, means, module, action, etc." are to be interpreted openly as referring to at least one instance of the element, apparatus, component, means, module, action, etc., unless explicitly stated otherwise.

The actions of any method disclosed herein do not have to be performed in the exact order disclosed, unless explicitly stated.

### BRIEF DESCRIPTION OF THE DRAWINGS

The inventive concept is now described, by way of example, with reference to the accompanying drawings, in which:
Fig. 1 is a schematic diagram illustrating a system and devices according to embodiments;
Figs. 2, 3, 4 and 5 are flowcharts of various aspects of methods according to embodiments;
Fig. 6 is a flowchart of various embodiments of a method;
Fig. 7 is a schematic diagram showing functional units of a device according to an embodiment;
Fig. 8 is a schematic diagram showing functional modules of a device according to an embodiment;
Fig. 9 shows one example of a computer program product comprising computer readable means according to an embodiment;
Fig. 10 is a flowchart of various embodiments of a method in a system.

### DETAILED DESCRIPTION

The inventive concept will now be described more fully hereinafter with reference to the accompanying drawings, in which certain embodiments of the inventive concept are shown. This inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description. Any action or feature illustrated by dashed lines should be regarded as optional.

Briefly, in contrast to the existing XR content filtering solutions, the present disclosure provides means and methods for determining the degree of cognitive load as a criterion for adapting information overlayed XR glasses, e.g., in order to avoid accidents. Further, methods and means are provided for dynamically decide what overlay information should be shown in a user's Field of View (FoV), peripheral view and/or non-XR devices in dependence on factors such as, for instance, policies, cognitive load and information priority. Still further, methods and means are provided for classifying unknown content, to alter or displace content before rendering it or adapting the currently displayed content if a policy is updated.

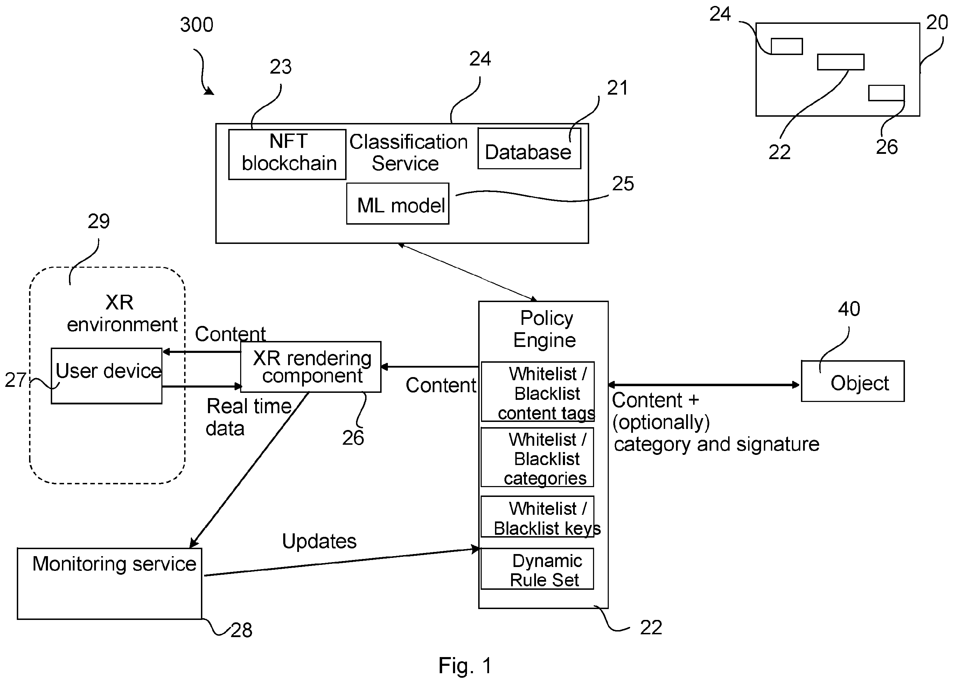

Fig. 1 is a schematic diagram illustrating a system 300 and devices according to embodiments. In a typical use case, a user utilizes a user device 27, e.g., XR glasses, XR headset, see-through glasses, mobile phone, tablet, hologram projector or smart lenses, in order to interact with an XR environment 29. The user device 27 may be presented with XR content (also known as overlay content) handled by external and/or internal entities: a policy engine 22, a classification service 24, an XR rendering component 26, and a monitoring service 28. The user device 27 may comprise one or more of these entities 22, 24, 26, 28. For instance, the classification service 24, the rendering component 26 and the policy engine 22 may be part of the user device 27, while the monitoring service 28 may be implemented e.g., in a cloud environment. In one embodiment, the user device 27 comprises all the entities 22, 24, 26, 28, in another embodiment, the user device 27 comprise entities 22, 24 and 26. The XR content may be provided to the user device 27 from different objects 40 and/or different vendors. When the user device 27 is presented with the overlay content, a policy engine 22 (e.g., located within the user device 27) decides if the information is trusted (e.g., previously approved or is received from a trusted vendor), distrusted (previously denied or by an untrusted vendor) or unknown. In embodiments wherein the policy engine 22 is not part of the user device 27, the user device 27 is presented with overlay content already handled by the policy engine 22.

In a generalized set of embodiments, illustrated at uppermost, right part of figure 1, a device 20 is provided comprising one or more of the described entities 22, 24, 26, 28. That is, the device 20 does not need to be the user device 27, but may instead be a separate device that a conventional user device (e.g., XR glasses) may connect to. In the illustrated case, the device 20 comprises the classification service 24, the rendering component 26 and the policy engine 22. Means for enabling connection to an user device 27, such as the XR glasses, is then also included in the device 20. Connecting means to Internet, cloud computing devices etc. may also be provided. Such connecting means may be devices for communication over any type of wireless (or wired) communication protocol(s).

The classification service 24 may be utilized to define how not yet consumed content should be classified. In the case where the content classification is unknown, the content payload (e.g., a 3D item, image, a text, an audio recording or a video) is sent to be categorized by the classification service 24. The classification service 24 returns the most likely categories and their respective confidence value to the policy engine 22.

Based on the obtained information, the policy engine 22 decides what content is allowed to be rendered in the user-perceived reality and how they may be rendered (e.g., in FoV or in peripheral vision). The policy engine 22 may filter content to only render content deemed prioritized while not rendering unwanted information. The policy engine 22 may also instruct the XR rendering component 26 to alter and move content to reduce impact on user's cognitive load.

The policy engine 22 comprises a set of rules which e.g., may have a minimum required confidence level for one or several content categories and will only render content if the confidence level exceeds these thresholds. The set of rules is continuously updated and may be defined manually by the user, defined by historical interactions, current context and cognitive load, or any combinations thereof.

The policy engine 27 may store content tags and confidence values from the classification service to speed up future classification of content
The system 300 thus comprises a classification service 24, a policy engine 22, a monitoring service 28 and an XR rendering component 26. The system 300 may be a single device comprising some or all of these entities 24, 22, 28, 26. In other embodiments, the system 300 is distributed, wherein some or all entities are located in, for instance, a cloud environment. The mentioned entities 24, 22, 28, 26 of the system 300 will be described more in detail in the following.

The classification service 24 comprises one or more classification tools for determining a respective appropriate category for different types of incoming content. The classification service 24 may, for instance, comprise machine learning algorithms for text, image, speech and/or object detection. These algorithms may be based on techniques such as convolutional neural networks (CNN), or other types of Deep Neural Networks (DNN), such as Long Short Term Memory Networks (LSTM), Recurrent Neural Networks (RNN), Autoencoders and Random Forest. The classification service 24 may apply several different algorithms or the same algorithm trained with different datasets, in order to increase reliability and/or credibility of the classification. The algorithms take the content data or a part of the content data as input and provide as output which class(es) the particular content may adhere to. The output class(es) may in turn be classified to more specific classes by additional algorithms.

The classification service 24 may use one or more of the above-described tools and set a list of categories and confidence values for each category based, for instance, on a weighted result.

An example on how to implement the classification service 24 is a convolutional neural network, which receives an image and a number of classes with a corresponding trust level. For instance, (Cat - 0.8, Dog - 0.1, Wolf 0.1), wherein the image comprising a cat has a trust level of 0.8 etc. The combination of classes is fed to another classification algorithm, e.g., Random Forest, which determines what age the content is suitable for. The second algorithm outputs a second classification: Child Friendly 0.99. The combination of (Child Friendly - 0.99, Cat - 0.8, Dog - 0.1, Wolf - 0.1) may then be supplied to the user device.

The classification service 24 may comprise one or more searchable databases 21 in which content tags are stored together with classifications and confidence values. The confidence values may not only reflect the probability of content being of a certain category but also how high confidence the information source, which provided the classification has. The classification service 24 may supply a content tag to search the database 21 for any stored categorizations.

In various embodiments, the database 21 may be updated by results of the machine learning algorithms described earlier. The database 21 may contain both global knowledge but also user-specific entries, such as personal data. The database 21 may be updated by human interactions, e.g., a user or another trusted human operator manually submitting classifications and trust level.

The classification service 24 may additionally (or alternatively) implement Non-fungible token (NFT)-based classification, utilizing a blockchain capable of handling NFTs for categorization. The content of the blockchain is publicly available and is useful when a user device 27 in an XR environment 29 is presented with content. In this context the objects/vendor(s) present the unique identity of the NFT associated with the content the object/vendor intends to deliver. The classification service 24 may utilize the blockchain in order to locate a picture or video and metadata bound to the NFT identity. In this context, the metadata may consist of classification data or tags. The classification service 24 may also verify who created an asset and whom is currently in possession of it. An exemplary scenario may be that a design bureau creates, advertises and sells the asset to a company. When they sell the asset in the shape of an NFT they transfer it to a wallet owned by the buying company. Allowing the classification service to verify who is in possession of the asset to be presented gives further possibilities to assess the credibility of the owning entity.

If the classification service 24 fails to detect what class the content belongs to, the content may be sent to a human operator for manual classification of the content. The classification service 24 may have the address (for instance email addresses or Internet Protocol, IP, addresses) of one or several devices able to receive requests for categorizing content. In some embodiments, this may also be the preferred classification method, where a parent or other trusted part must allow content for children in the XR environment 29.

As is clear by the various examples given, the classification service 24 may be implemented in many different ways. Besides the described examples of using machine learning based classification, database-based classification, NFT-based classification and manual classification (and combinations thereof), various other methods may be used.

Next, the policy engine 22 is described in more detail. The policy engine 22 is a component with a function to decide if and how the content should be rendered for the user. A configuration of the policy engine 22 may have a permanent part comprising user prerequisites such as gender, age, etc.; a semi-permanent part comprising user preferences such as shopping list, preferred ads, etc.; and a temporary part which is a continuously updated part in order to reflect the susceptibility to new content and the current cognitive load of the user. These factors may be combined for keeping and continuously updating a current set of rules for how to handle incoming content.

As another, more specific example of user preferences that may be stored in the semi-permanent part, cognitive load can be mentioned. The settings for the cognitive load may set such that they prevent e.g., non-relevant content to be shown to a service technician that currently is in a stressful situation. Yet another example is the use of an in-car head up display, on which content to be shown may be based on speed, darkness, time, etc. based on set user preferences. Still another example is adapting XR user interface according to settings: a user with XR glasses sees a restaurant, and the content presented to her may comprise menu, ranking, price indications, waiting time. The content may be adapted dynamically, e.g., if the user is short of time (which can be decided based on an available calendar/schedule), only dishes that can be provided rapidly are shown. Another example is use in a hospital; e.g., during an operation one or more mistakes are made, upon which the cognitive load of the surgeon most likely increases, then the policy engine may prioritize what information to show for the supposedly highly stressed (cognitive overload) personnel.

From the above it is realized that the various aspects of the present teachings may be implemented for and adapted to various situations.

Input to the policy engine 22 comprises both external overlay content, data from cognitive load monitoring service and data from classification service. Such data is processed by the policy engine 22 for various purposes, for instance: to determine overlay content priority and if said content may be modified by reducing and/or displacing the content; to analyze the cognitive load of the user and susceptibility to new content; and to evaluate historical data and determine whether present context or certain task may impact a user's focus.

The policy engine 22 contains a set of rules comprising one or several rules which applies to one or several content categories. The rules may comprise one or several categories, a rendering decision and optionally a threshold. The threshold may indicate a minimum confidence level (e.g., between 0 and 1) that the content classification needs to fulfill for the rendering decision to apply. As an example, the user only wants coffee offers to be shown if the confidence value is above 0.8. If the confidence value is below this set threshold, the coffee offers will not be shown to the user, even if the category/vendor is approved (whitelisted, described later).

As another example, the category "child-friendly" may be used in order to filter out content that is unsuitable for children and may be set with a 0.99 threshold value to ensure that no false negatives get through, i.e., to ensure that the children do not get to see the unsuitable content.

As another, more advanced example, the policy may be set in dependence on, for instance, the amount of content currently rendered for the user, its priority, the user's cognitive load and an assessed concentration needed for a certain task. Exemplary policy may then be set to:
- allow all content to be rendered in the XR glasses FoV (when amount of content, data priority, cognitive load and type of context combined are below a balance/ratio score),
- reduce amount of content rendered in the XR glasses FoV (when amount of content exceeds a level high, priority data is varying, cognitive load is medium, type of context is demanding),
- overlay data is scaled down and rendered in the visual periphery of the XR glasses (when amount of content is normal, data priority is high, cognitive load is high, type of context is normal)

The process of dynamic rendering will enable a user, having both hands occupied, like a service technician, surgeon etc., to have relevant information available to execute a task in a secure and efficient manner. The policy manages the balance/ratio score related to input data, user cognitive load and context/task type.

The policy engine 22 may additionally store one or several white-/gray-/blacklists for previously seen content and/or objects. This allows the policy engine 22 to "remember" content known to be good or bad, which speeds up future rendering. Graylists may be used to list vendors which have previously provided content, but which are not yet classified as being known as good (i.e., on whitelists) or being known as bad (i.e., on blacklists). These lists may be updated based on output from the classification service 24.

Next, the monitoring service 28 arranged to monitor the cognitive load is described in more detail. The monitoring service 28 interprets the current state and context of the user based on data that it receives, e.g., from a XR rendering component 26. The cognitive load can be seen as how preoccupied the user is, either with XR content or with real-life objects, or both. The user device 27 may be arranged to measure and/or register various data relating to the user, e.g., by tracking the users' pupil diameter, gaze etc. using, for instance, sensors. The XR rendering component 26 may then supply these values to the monitoring service 28, and the monitoring service 28 may utilize these values for determining if the users' cognitive load is above a threshold.

Susceptibility to new content is related to the cognitive load and is a measure on how much content a user is able to handle in a certain situation. The susceptibility may also be measured by sensors, such as e.g., camera and velocity sensor. The monitoring service 28 may supply data on the users' cognitive load to an algorithm and determine if the user is in a situation in which distracting content is unsuitable, for instance when driving a car.

The monitoring service 28 continuously supplies values indicating current cognitive load and current content susceptibility to the policy engine 22, which in turn updates its temporary configuration and thereby its set of rules accordingly.

Finally, the XR rendering component 26 is described in more detail. The XR rendering component 26 is responsible for presenting the overlay content in a manner decided by the policy engine 22. The XR rendering component 26 receives content from the policy engine 22 and may also receive instructions to alter, displace or remove currently rendered content. It may also track certain real time data indicating cognitive load (e.g., data such as pupillary diameter and number of gaze fixations) susceptibility to new material (e.g., data on environment and velocity).

The system 300 is described to comprise a classification service 24, a policy engine 22, a monitoring service 28 and an XR rendering component 26 but may be implemented in various ways. As noted earlier, the system 300 may be a single device comprising some or all of these entities 24, 22, 28, 26, or the system 300 may be distributed, wherein some or all entities are located in, for instance, a cloud environment.

In a preferred embodiment, the entities 22, 24, 26, 28 of the system 300 are placed within a single device.

In other embodiments, the system 300 may be a distributed system, wherein the entities 22, 24, 26, 28 may be distributed over several devices controlled by the same user.

For instance, as a particular example the classification service 24 may be embodied as a service within a smartphone, the cognitive load monitoring service 28 and policy engine 22 may be embodied within a smartwatch and the XR rendering component 26 may be a placed in a pair of XR glasses. In such an embodiment, the policy engine 22 may additionally comprise a set of currently active user devices, such as a smartphone or a smartwatch. Content which is not rendered for the user may instead be displayed on a screen of a non-XR user device. This may, for instance, be an alternative when the user's cognitive load is high and new content should not be presented in front of the user.

From the description hitherto, the various embodiments can be summarized as comprising three different phases: receival, classification and rule fulfillment. During all of these phases, the XR rendering component may measure indication of the user's cognitive load (pupil diameter, gaze etc.) and supply these to cognitive load monitoring service 28. The monitoring service 28 returns an indication of the current cognitive load and current susceptibility to new content. This is weighed in as a factor in the rule fulfillment phase.

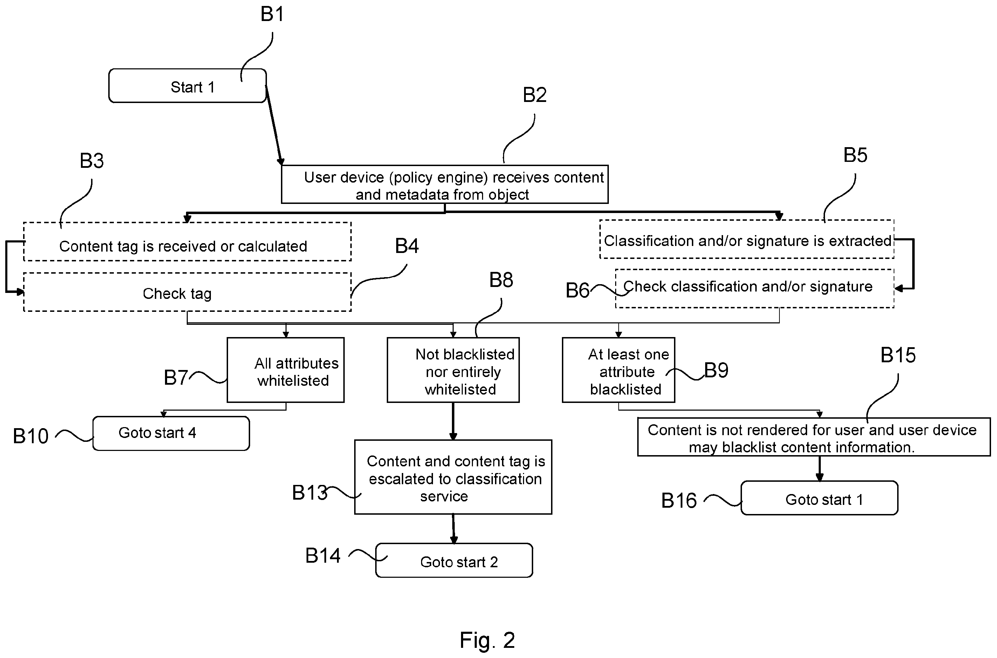

Fig. 2 is a flowchart of various aspects of methods according to embodiments. In particular, the flowchart describes a receival phase. It is noted that dotted lines indicate optional steps. In the receival phase, a user device 27 in an XR environment 29 is presented with overlay content from objects in proximity, the content is received by the policy engine 22. Examples of such objects may include objects of common interest, such as stores, tourist attractions and billboards but also objects of interest to specific individuals such as vehicles, buildings and industrial equipment. The content may comprise images, text, animations, 3D items etc. and may be supplied together with placement and size information. The content is optionally accompanied with metadata such as a classification, a signature and/or a content tag.

The flow starts in box B1, and continues to box B2, in which a policy engine 22 receives content and (in this case) metadata from an object. From box B2, there are two similar optional flows shown, a first optional flow from box B2 to boxes B3, B4, and a second optional flow from box B2 to boxes B5, B6.

In the first optional flow, in box B3, a content tag is received or calculated, e.g., by hashing the content. From box B3, flow continues to box B4, in which the content tag is checked against any blacklist(s) and/or whitelist(s).

In the second optional flow, in box B5, a classification and/or signature is extracted. From box B5, flow continues to box B6, in which the classification and/or signature are checked against any blacklist(s) and/or whitelist(s).

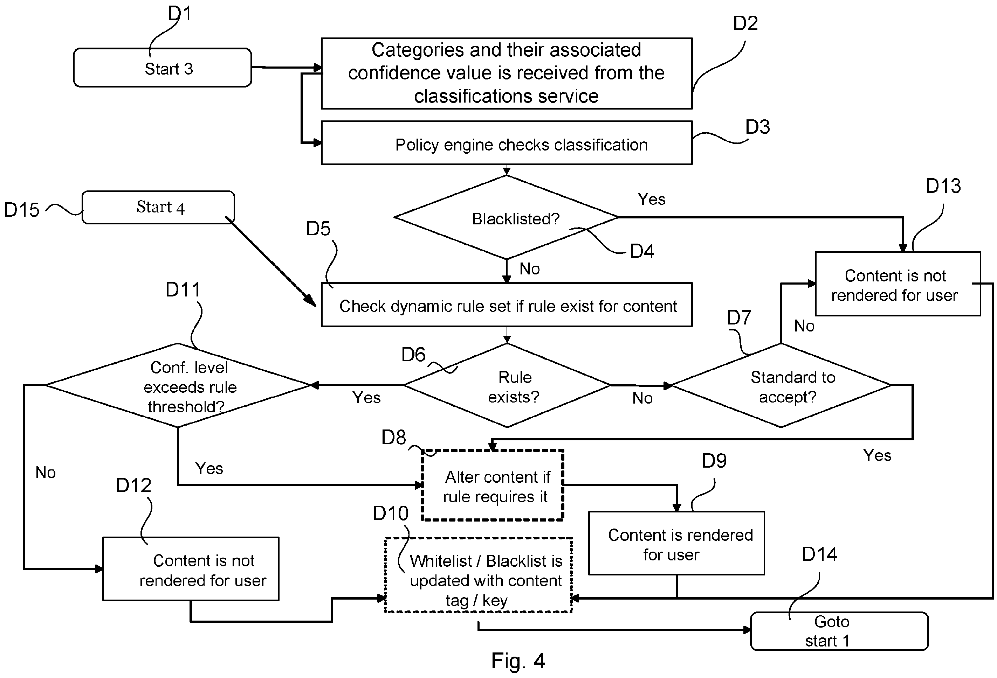

Flows then continues to three different flows, representing three different cases. In a first of these flows, the flow starting in box B7, all attributes are whitelisted. In this case, flow continues to box B10, from which flow continues to Start 4 (of figure 4), wherein rule fulfilment must be ensured.

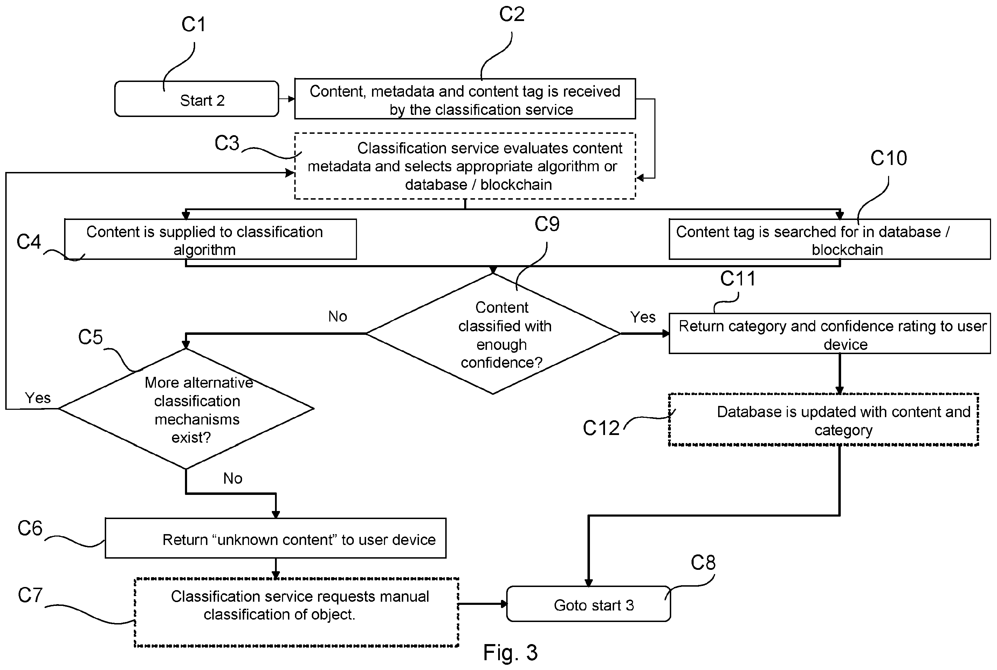

In a second of these flows, the flow starting in box B8, the attributes are nor blacklisted neither entirely whitelisted, instead, they may be seen as greylisted. Flow continues to box B13, in which the content and content tag are sent to the classification service, for being classified. Flow then continues to box B14, from which flow continues to Start 2 (of figure 3), wherein classification of content is made.

In a third of these flows, the flow starting in box B9, at least one attribute is blacklisted. Flow continues to box B15, and the content is not rendered for the user. Flow then continues to box B16, from which flow returns to box B1, wherein receival of new content can be made.

**Fig.** 3 is a flowchart of various aspects of methods according to embodiments. In particular, the flowchart describes the classification phase. The flow starts in box C1, and continues to box C2, in which the classification service 24 receives one or more of content, metadata and content tag. Flow continues to an optional box, box C3, in which the classification service 24 also evaluates the content and/or metadata in order to select an appropriate algorithm or database/blockchain for use. Flow then continues, either directly from box C2 or via box C3, to box C4 or box C10.

In box C4, the content is supplied to a classification algorithm (described earlier).

In box C10, the content tag is searched for in the database/blockchain (described earlier).

The flows from C4 and C10 both continue to box C9, wherein it is determined whether or not the content is classified with high enough confidence, for instance determining if a threshold requirement is met.

If the outcome of determination box C9 is that the content is indeed classified with high enough confidence, flow continues to box C11, in which category of content and confidence rating is provided to the user device 27. From box C11, flow continues to box C12, which is an optional box if there is a database, then the database is updated with the newly classified content and its category. Flow ends in box C8, wherein a new flow may begin in box D1 (of figure 4).

If the outcome of determination box C9 is instead that the content is not classified with high enough confidence, flow continues to box C5. In box C5 it is determined whether there are other, alternative classification mechanisms. If there is, flow returns to box C3 for attempting these alternative classifications. If the outcome of determination box C5 is "No", i.e., that there are no further classification alternatives, flow continues to box C6, in which the user device is informed that the content is unknown. Flow may then continue to an optional box C7, in which the classification service 24 requests the user to make a manual classification of the object. Flow ends in box C8, wherein a new flow may begin in box D1 (of figure 4).

**Fig.** 4 is a flowchart of various aspects of methods according to embodiments. In particular, the flowchart describes the rule fulfilment phase. The flow starts in box D1 and continues to box D2. In box D2, the classification service 24 conveys content categories and their respective associated confidence value. Flow continues to box D3, wherein the policy engine 22 checks the classification against blacklist(s), ensuring that no blacklisted content is rendered, e.g., that a child does not get to see violence. Flow then continues to decision box D4.

If, in decision box D4, the content is blacklisted, then flow continues to box D13, and the content is not rendered to the user. From box D13, flow continues to an optional box D10, in which any white- and/or blacklists are updated with content tag or content signature or the vendor's/object's public key. Flow then ends in box D14, from which a new flow may begin in box B1 (figure 2).

If, in decision box D4, the content is not on a blacklist, flow continues to box D5. In box D5, the content is checked against a dynamic rule set to see if (and how) any rule applies to the content. The rule set may be changed dynamically in dependence on, for instance, cognitive load, user context etc. The monitoring service 28 may continuously determine data related to the current environment and the state of the user and provide the data to policy engine 22. From box D5, flow continues to decision box D6, in which it is determined if there is or is not a rule for the content. If there is, flow continues to decision box D11, in which it is determined whether the content meets rule threshold(s) (e.g., a confidence threshold) set for the particular rule(s). If, for example, the confidence level for the content indeed exceeds the corresponding rule threshold, i.e., the outcome of decision box D11 is "yes" then flow continues to optional box D8, in which the content may be altered if a particular rule requires it. Flow then continues to box D9, wherein the content is rendered. Flow may go via box D10 (already described) before it ends in box D14, from which a new flow may begin in box B1 (figure 2).

If, in decision box D11, the outcome is "no", i.e., the confidence level for the rule is not met, then flow continues to box D12 and the content is not rendered to the user. Flow may go via the optional box D10 (already described) before it ends in box D14, from which a new flow may begin in box B1 (figure 2).

If, in decision box D6, the outcome is "no", i.e., there is no rule applying for the content, flow continues to decision box D7, wherein it is determined if the content is typically accepted, i.e., where the fallback decision is to accept the content, if nothing else has been specified. If no, flow continues to box D13, i.e., the content is not rendered for the user, and flow may go via box D10 (already described) before it ends in box D11, from which a new flow may begin in box B1 (figure 2). If instead, in decision box D7, the outcome is "yes", i.e., the content is typically accepted, flow continues to optional box D8 (already described), via box D9 and optional box D10 (both already described), before it ends in box D14, from which a new flow may begin in box B1 (figure 2).

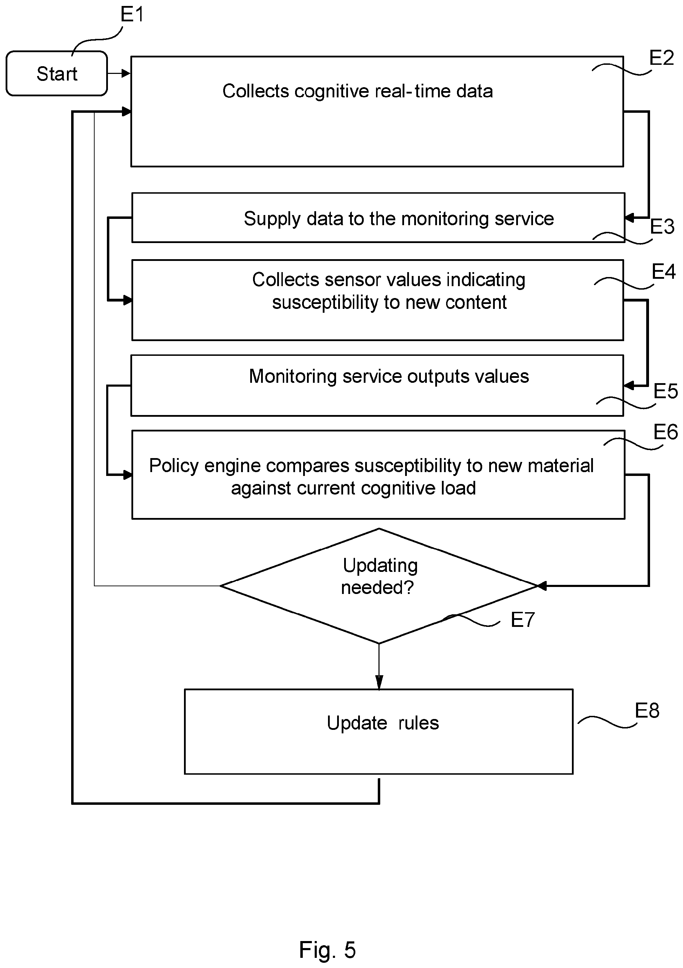

**Fig. 5** is a flowchart of various aspects of methods according to embodiments. In particular, the flowchart describes an example on how the policy can be continuously updated with regards to cognitive load.

Upon starting the policy updating, box E1, a user device collects, in box E2, cognitive data. The data is preferably real-time data, such as user's pupil diameter, gaze, pulse, heart rate etc. Flow continues to box E3, wherein the data is supplied to the monitoring service 28. Next, in box E4, the user device collects sensor values which are indicative for susceptibility to new content. Examples of such sensor values may, for instance, be movement speed, camera feed for environment (a camera placed such as to capture the environment), data for determining cognitive load, such as user context (time of day, task being performed, amount of data that is displayed etc.), biological data (heart rate, pupil size, motion patterns, ventilation rate, breathing rate, etc.), movements/task of user (sitting in a sofa, walking, running, driving a car, etc.) etc. Flow continues to box E5, wherein the monitoring service 28 provides, to the policy engine 22, values indicative for the cognitive load of the user and her susceptibility to new content. These values are based on the data collected in box E4 (as exemplified in relation to box E4). Next, in box E6, the policy engine 22 compares, for instance, susceptibility to new material with the user's current cognitive load. Flow continues to decision box E7, wherein the policy engine 22 determines whether there is a need for one or more rules in the dynamic rule set to be updated. Next, in box E8, the policy engine 22 is updated. The dynamic rule set may, for instance, comprise changing thresholds, requiring content to be altered before rendering, only allowing certain type of content or blocking all content.

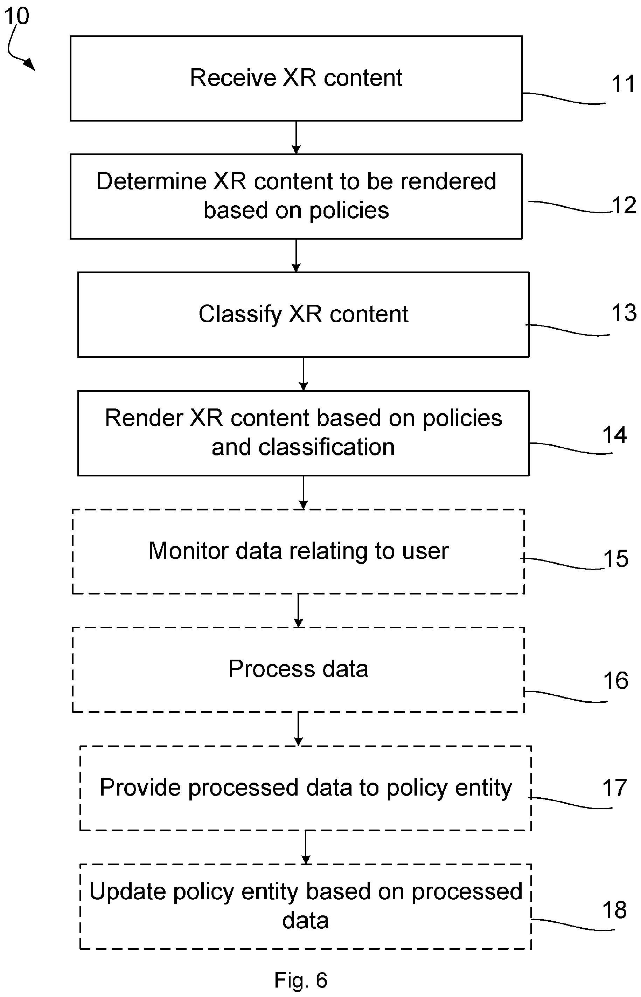

**Fig. 6** is a flowchart of various embodiments of a method for rendering Extended Reality, XR, content to a user. The method 10 may be performed in a device 20, such as, for instance, in the user device 27. In such embodiment, the user device 27 comprises the policy entity 22, the classification service 24 and the rendering component 26.

The method 10 comprises:
- receiving 11, in the device 20, XR content,
- determining 12, in a policy entity 22, the XR content to be rendered based on one or more policies,
- classifying 13, in a classification service 24, the XR content, and
- rendering 14 the XR content in an XR environment based on the one or more policies and the classification of the XR content.

The method 10 provides a number of advantages. For instance, the method 10 enables an XR experience that accounts for, and reduces, risks of personal injuries, as have been exemplified earlier. By accounting for and using factors such as, for instance, various policies and classifications, cognitive load, proximity to objects and direction of user in the information filtering, the personal safety during XR events is improved. Further, XR content may be altered and/or displaced before rendering it if a policy is updated, or the currently displayed content may be adapted if a corresponding policy is updated. These aspects enable decisions to be made dynamically based on various factors (examples of which have been given earlier), which factors may change during the XR experience.

Further, the method 10 may be used in a number of scenarios, and in particular in virtual environments or in a real environment with augmented content presented as an overlay. In such scenarios, a high amount of information may be presented in e.g., XR glasses and the risk of cognitive overload is then high. For instance, when a user is unable or unsuited to manually interact with the content in the XR environment, e.g., when driving a car or performing tasks wherein both hands are occupied, the method 10 reduces risk of personal injuries happening by, for instance, rendering less amount of content.

In an embodiment, the determining 12 of the XR content is performed dynamically during an XR event in the XR environment. This has been described earlier, and such embodiments enable adapting to an updated situation, such as the one exemplified in above paragraph. Continuing this example: if the user stops the car, the XR content may be updated to suit this new situation, in which the car does not move.

In an embodiment, the method 10 comprises monitoring 15, in the monitoring service 28, data relating to the user. The monitoring 15 may be performed after the rendering 14, and comprises in an embodiment:
- processing 16, in the monitoring service 28, the data,
- providing 17 the processed data to the policy entity 22, and
- updating 18 the policy entity 22 based on the processed data.

In variations of the above embodiment, the determining 12 is, at least partly, made based on the data provided by the monitoring service 28.

In various embodiments, the rendering 14 comprises determining whether to render particular XR content based on determined current cognitive load of the user.

In variations of the above embodiment, the current cognitive load of the user is determined continuously or intermittently, the cognitive load being determined based on one or more of: user location, user activity level, biometrics data, movement pattern of user and user's type of activity.

In various embodiments, the determining 12 is based on one or more of: trustworthiness of the XR content, user reaching a threshold for user cognitive load, threshold for user susceptibility to XR content in view of current context and type of task, user's location, user's type of activity, user preferences, historical behaviour of user interacting with other users, white-/blacklisted content, white-/blacklisted content metadata, white-/blacklisted cryptographical additions to the content, estimated relevance to the user, activity level of user, user being stationary, at rest or in motion, user being in a vehicle, user being outdoors or indoors, time of day. It is noted that any combination of these factors (as well as others) may be applied.

In various embodiments, the classifying 13 comprises determining one or more of: a category to which the XR content belongs, a confidence value for at least one specific category and a recommendation for future content with same origin.

In various embodiments, the classification service 24 comprises one or more of: an image detection algorithm, a text detection algorithm, an object detection algorithm, a database of content and respective related classification, non-fungible tokens with metadata stored on a blockchain, a speech detection algorithm.

In various embodiments, the device 20 is or is included in a user device selected among: XR glasses, smart lenses, smart phone, tablet, laptop, hologram projectors, any content overlay-enabled screen, head-up display, see-through display.

A device 20 is also disclosed, the device 20 being suitable for providing Extended Reality, XR, content to a user. The device 20 may be configured to perform any of the embodiments of the method 10 as described herein. In an embodiment the device 20, for instance the user device 27, is configured to:
- receive XR content,
- determine which XR content to render based on one or more policies,
- classify the XR content,
- render XR content in an XR environment based on the one or more policies and the classification of the XR content.

In an embodiment, the device 20 is configured to determine the XR content dynamically during an XR event in the XR environment.

In various embodiments, the device 20 is configured to:
- monitor, in a monitoring service 28, data relating to the user,
- process, in the monitoring service 28, the data,
- provide the processed data to the policy entity 22, and
- update the policy entity 22 based on the processed data.

In a variation of the above embodiment, the device 20 is configured to determine, at least partly, based on the data provided by the monitoring service 28.

In various embodiments, the device 20 is configured to determine whether to render particular XR content, based on a determined current cognitive load of the user.

In various embodiments, the device 20 is configured to determine the current cognitive load of the user continuously or intermittently, wherein the cognitive load is determined based on one or more of: user location, user activity level, biometrics readouts, movement pattern of user and user's type of activity.

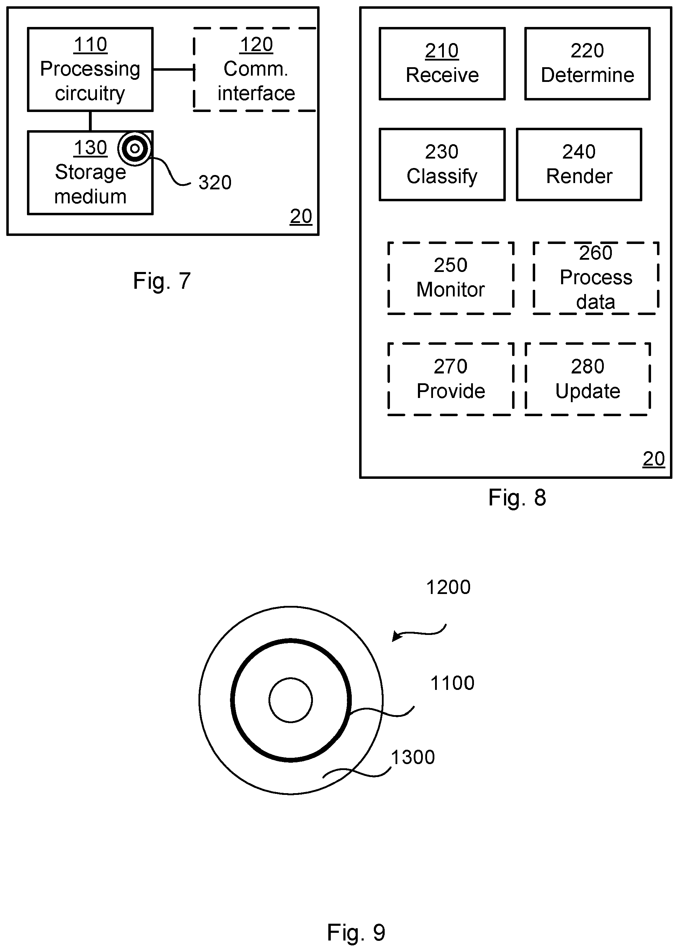

**Fig. 7** schematically illustrates, in terms of a number of functional units, the components of a device 20 according to an embodiment. Processing circuitry 110 is provided using any combination of one or more of a suitable central processing unit (CPU), multiprocessor, microcontroller, digital signal processor (DSP), etc., capable of executing software instructions stored in a computer program product 1200 (as in Fig. 9), e.g., in the form of a storage medium 130. The processing circuitry 110 may further be provided as at least one application specific integrated circuit (ASIC), or field programmable gate array (FPGA).

Particularly, the processing circuitry 110 is configured to cause the device 20 to perform a set of operations, or actions, as disclosed above. For example, the storage medium 130 may store the set of operations, and the processing circuitry 110 may be configured to retrieve the set of operations from the storage medium 130 to cause the device 20 to perform the set of operations. The set of operations may be provided as a set of executable instructions. The processing circuitry 110 is thereby arranged to execute methods as herein disclosed.

The storage medium 130 may also comprise persistent storage, which, for example, can be any single one or combination of magnetic memory, optical memory, solid state memory or even remotely mounted memory.

The device 20 may further comprise a communications interface 120 for communications with other entities, functions, nodes, and devices, over suitable interfaces. As such the communications interface 120 may comprise one or more transmitters and receivers, comprising analogue and digital components.

The processing circuitry 110 controls the general operation of the device 20 e.g., by sending data and control signals to the communications interface 120 and the storage medium 130, by receiving data and reports from the communications interface 120, and by retrieving data and instructions from the storage medium 130. Other components, as well as the related functionality, of the device 20 are omitted in order not to obscure the concepts presented herein.

**Fig. 8** schematically illustrates, in terms of a number of functional modules, the components of a device 20 according to an embodiment. The device 20 of Fig. 8 comprises a number of functional modules; a receive module 210 configured to receive XR content, a determine module 220 configured to determine XR content to be rendered based on one or more policies, a classify module 230 configured to classify the XR content, and a render module 240 configured to render XR content based on policies and classification of XR content. The device 20 of Fig. 8 may further comprise a number of optional functional modules, such as any of a monitor module 250 configured to monitor data relating to user, a process module 260 configured to process data, a provide module 270 configured to provide processed data to policy entity, and an update module 280 configured to update policies based on processed data. In general terms, each functional module 210 - 280 may be implemented in hardware or in software. Preferably, one or more or all functional modules 210 - 280 may be implemented by the processing circuitry 110, possibly in cooperation with the communications interface 120 and the storage medium 130. The processing circuitry 110 may thus be arranged to from the storage medium 130 fetch instructions as provided by a functional module 210 - 280 and to execute these instructions, thereby performing any actions of the device 20 as disclosed herein.

**Fig. 9** shows one example of a computer program product 1200 comprising computer readable means 1300. On this computer readable means 1300, a computer program 1100 can be stored, which computer program 1100 can cause the processing circuitry 110 and thereto operatively coupled entities and devices, such as the communications interface 120 and the storage medium 130, to execute methods according to embodiments described herein. The computer program 1100 and/or computer program product 1200 may thus provide means for performing any actions of the device 20 as herein disclosed.

In the example of Fig. 9, the computer program product 1200 is illustrated as an optical disc, such as a CD (compact disc) or a DVD (digital versatile disc) or a Blu-Ray disc. The computer program product 1200 could also be embodied as a memory, such as a random access memory (RAM), a read-only memory (ROM), an erasable programmable read-only memory (EPROM), or an electrically erasable programmable read-only memory (EEPROM) and more particularly as a non-volatile storage medium of a device in an external memory such as a USB (Universal Serial Bus) memory or a Flash memory, such as a compact Flash memory. Thus, while the computer program 1100 is here schematically shown as a track on the depicted optical disk, the computer program 1100 can be stored in any way which is suitable for the computer program product 1200.

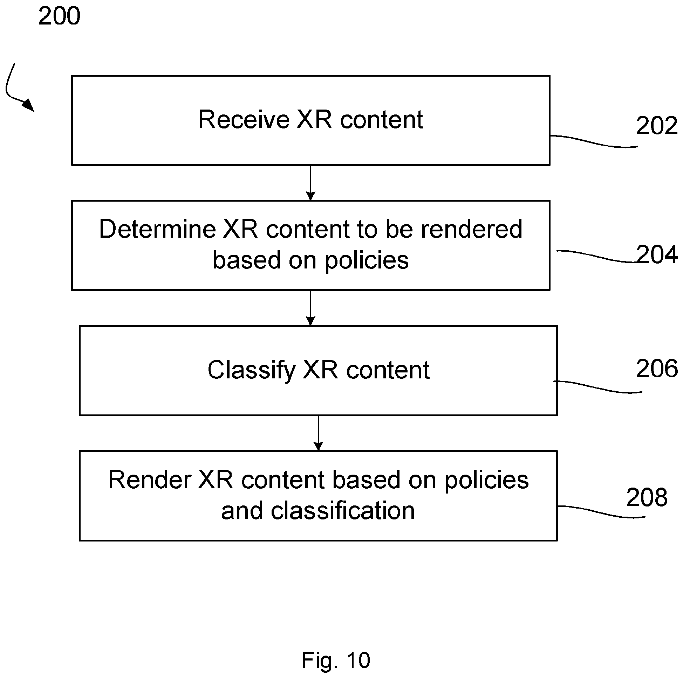

**Fig. 10** is a flowchart of various embodiments of a method in a system. A method 200 is provided for rendering Extended Reality, XR, content to a user, wherein the method 200 is performed in a system 300. The system 300 comprises, in an embodiment, the earlier described entities: the policy engine 22, the classification service 24, the XR rendering component 26 and the monitoring service 28. The method 200 comprises receiving 202 XR content. This step may, for instance, be performed in the XR rendering component 26. The method 200 comprises determining 204 the XR content to be rendered based on one or more policies. This step may be performed in the policy engine 22. The method 200 comprises classifying 206 the XR content. This step may be performed in the classification service 24. The method 200 comprises rendering 208 the XR content in an XR environment based on the one or more policies and the classification of the XR content. This step may be performed in the XR rendering component 26.

In an embodiment, at least one step is performed in a cloud computing environment. For instance, in some embodiments one or both of determining 204 and classifying 206 are performed in such a cloud computing environment.

A system 300 for rendering Extended Reality, XR, content to a user is also provided. The system 300 is configured to perform any of the embodiments of the method 200 as described. For instance, in one embodiment, one or both of determining and classification are performed in a cloud computing environment.

The system 200 may be configured to perform any of the steps of the method 10 in the device 20, but in a distributed manner. These embodiments are not repeated here, as several examples on how to implement the device has been given, and examples on which entities may be external to the device 20.

The inventive concept has mainly been described above with reference to a few embodiments. However, as is readily appreciated by a person skilled in the art, other embodiments than the ones disclosed above are equally possible within the scope of the inventive concept, as defined by the appended patent claims.

## Claims

1. A method (10) for rendering Extended Reality, XR, content to a user, the method (10) being performed in a device (20), and comprising:
- receiving (11), in the device (20), XR content,
- determining (12), in a policy entity (22), the XR content to be rendered based on one or more policies,
- classifying (13), in a classification service (24), the XR content, and
- rendering (14) the XR content in an XR environment based on the one or more policies and the classification of the XR content, the method being **characterized in that**: the determining (12) is based at least on a threshold for user susceptibility to XR content in view of current context and type of user task, being the user susceptibility value measured by sensors.

2. The method (10) as claimed in claim 1, wherein the determining (12) the XR content is performed dynamically during an XR event in the XR environment.

3. The method (10) as claimed in claim 1 or 2, comprising
- monitoring (15), in a monitoring service (28), data relating to the user,
- processing (16), in the monitoring service (28), the data,
- providing (17) the processed data to the policy entity (22), and
- updating (18) the policy entity (22) based on the processed data.

4. The method (10) as claimed in claim 3, wherein the determining (12) is, at least partly, made based on the data provided by the monitoring service (28).

5. The method (10) as claimed in any of the preceding claims, wherein the rendering (14) comprises determining whether to render particular XR content based on determined current cognitive load of the user, wherein the current cognitive load of the user is determined continuously or intermittently, the cognitive load being determined based on one or more of: user location, user activity level, biometrics data, movement pattern of user and user's type of activity.

6. The method (10) as claimed in any of the preceding claims, wherein the determining (12) is based on one or more of: trustworthiness of the XR content, user reaching a threshold for user cognitive load, user's location, user's type of activity, user preferences, historical behaviour of user interacting with other users, white-/blacklisted content, white-/blacklisted content metadata, white-/blacklisted cryptographical additions to the content, estimated relevance to the user, activity level of user, user being stationary, at rest or in motion, user being in a vehicle, user being outdoors or indoors, time of day.

7. The method (10) as claimed in any of the preceding claims, wherein the classifying (13) comprises determining one or more of: a category to which the XR content belongs, a confidence value for at least one specific category and a recommendation for future content with same origin.

8. The method (10) as claimed in any of the preceding claims, wherein the classification service (24) comprises one or more of: an image detection algorithm, a text detection algorithm, an object detection algorithm, a database of content and respective related classification, non-fungible tokens with metadata stored on a blockchain, a speech detection algorithm.

9. A computer program (100) for rendering Extended Reality, XR, content to a user, the computer program comprising computer code which, when run on processing circuitry (110) of a device (20), causes the device (20) to:
- receive the XR content,
- determine the XR content to be rendered based on one or more policies,
- classify the XR content, and
- render the XR content in an XR environment based on the one or more policies and the classification of the XR content,
wherein the XR content to be rendered is determined based at least on a threshold for user susceptibility to XR content in view of current context and type of user task, being the user susceptibility value measured by sensors.

10. A device (20) for providing Extended Reality, XR, content to a user, the device being configured to:
- receive XR content,
- determine which XR content to render based on one or more policies,
- classify the XR content,
- render XR content in an XR environment based on the one or more policies and the classification of the XR content,
wherein the XR content to be rendered is determined based at least on a threshold for user susceptibility to XR content in view of current context and type of user task, being the user susceptibility value measured by sensors.

11. The device (20) as claimed in claim 10, configured to determine the XR content dynamically during an XR event in the XR environment.

12. The device (20) as claimed in claim 10 or 11, configured to:
- monitor, in a monitoring service (28), data relating to the user,
- process, in the monitoring service (28), the data,
- provide the processed data to the policy entity (22), and
- update the policy entity (22) based on the processed data.

13. The device (20) as claimed in claim 12, configured to determine, at least partly, based on the data provided by the monitoring service (28).

14. The device (20) as claimed in any of claims 10 - 13, configured to determine whether to render particular XR content, based on determined current cognitive load of the user.

15. The device (20) as claimed in claim any of claims 10 - 14, configured to determine the current cognitive load of the user continuously or intermittently, wherein the cognitive load is determined based on one or more of: user location, user activity level, biometrics readouts, movement pattern of user and user's type of activity.

16. A method (200) for rendering Extended Reality, XR, content to a user, the method (200) being performed in a system (300) and comprising:
- receiving (202) XR content,
- determining (204) the XR content to be rendered based on one or more policies,
- classifying (206) the XR content, and
- rendering (208) the XR content in an XR environment based on the one or more policies and the classification of the XR content,
the method being **characterized in that**:
the determining (12) is based at least on a threshold for user susceptibility to XR content in view of current context and type of user task, being the user susceptibility value measured by sensors; and
wherein at least one of the method steps is performed in a cloud computing environment.

## Patentansprüche

1. Verfahren (10) zum Rendern von Extended-Reality-, XR-, Inhalt für einen Nutzer, wobei das Verfahren (10) in einer Vorrichtung (20) durchgeführt wird und umfasst:
- Empfangen (11), in der Vorrichtung (20), von XR-Inhalt,
- Bestimmen (12), in einer Richtlinieneinheit (22), des XR-Inhalts, der basierend auf einer oder mehreren Richtlinien gerendert werden soll,
- Klassifizieren (13), in einem Klassifizierungsdienst (24), des XR-Inhalts, und
- Rendern (14) des XR-Inhalts in einer XR-Umgebung basierend auf einer oder mehrerer Richtlinien und der Klassifizierung des XR-Inhalts, wobei das Verfahren **dadurch gekennzeichnet ist, dass**:
das Bestimmen (12) mindestens auf einem Schwellenwert für die Empfänglichkeit des Nutzers gegenüber XR-Inhalt im Hinblick auf einen aktuellen Kontext und eine Art einer Nutzeraufgabe basiert, wobei der Wert der Empfänglichkeit des Nutzers durch Sensoren gemessen wird.

2. Verfahren (10) nach Anspruch 1, wobei das Bestimmen (12) des XR-Inhalts dynamisch während eines XR-Ereignisses in der XR-Umgebung durchgeführt wird.

3. Verfahren (10) nach Anspruch 1 oder 2, umfassend
- Überwachen (15), in einem Überwachungsdienst (28), von Daten in Bezug auf den Nutzer,
- Verarbeiten (16) der Daten im Überwachungsdienst (28),
- Bereitstellen (17) der verarbeiteten Daten der Richtlinieneinheit (22), und
- Aktualisieren (18) der Richtlinieneinheit (22) basierend auf den verarbeiteten Daten.

4. Verfahren (10) nach Anspruch 3, wobei das Bestimmen (12) mindestens teilweise basierend auf vom Überwachungsdienst (28) bereitgestellten Daten erfolgt.

5. Verfahren (10) nach einem der vorstehenden Ansprüche, wobei das Rendern (14) Bestimmen umfasst, ob bestimmter XR-Inhalt basierend auf der bestimmten aktuellen kognitiven Belastung des Nutzers gerendert werden soll, wobei die aktuelle kognitive Belastung des Nutzers kontinuierlich oder intermittierend bestimmt wird und die kognitive Belastung basierend auf einem oder mehr bestimmt wird von: Nutzerstandort, Nutzeraktivitätsniveau, biometrischen Daten, Bewegungsmuster eines Nutzers und Art der Nutzeraktivität.

6. Verfahren (10) nach einem der vorstehenden Ansprüche, wobei das Bestimmen (12) auf einem oder mehr basiert von: Vertrauenswürdigkeit des XR-Inhalts, Erreichen durch den Nutzer eines Schwellenwerts der kognitiven Belastung des Nutzers, Nutzerstandort, Art der Nutzeraktivität, Nutzerpräferenzen, bisherigem Interaktionsverhalten des Nutzers mit anderen Nutzern, Inhalt auf der Whitelist/Blacklist, Metadaten zu Inhalten auf der Whitelist/Blacklist, kryptografische Ergänzungen des Inhalts auf der Whitelist/Blacklist, geschätzte Relevanz für den Nutzer, Aktivitätsniveau des Nutzers, ob der Nutzer stationär, in Ruhe oder in Bewegung ist, ob sich der Nutzer in einem Fahrzeug befindet, ob sich der Nutzer im Freien oder in Innenräumen befindet, Tageszeit.

7. Verfahren (10) nach einem der vorstehenden Ansprüche, wobei die Klassifizierung (13) Bestimmen von einem oder mehr umfasst von: einer Kategorie, welcher der XR-Inhalt angehört, einem Konfidenzwert für mindestens eine bestimmte Kategorie und einer Empfehlung für künftigen Inhalt gleicher Herkunft.

8. Verfahren (10) nach einem der vorstehenden Ansprüche, wobei der Klassifizierungsdienst (24) eines oder mehr umfasst von: einem Bilderkennungsalgorithmus, einem Texterkennungsalgorithmus, einem Objekterkennungsalgorithmus, einer Datenbank mit Inhalt und der jeweils zugehörigen Klassifizierung, nicht-austauschbaren Token mit auf einer Blockchain gespeicherten Metadaten, einem Spracherkennungsalgorithmus.

9. Computerprogramm (100) zum Rendern von Extended-Reality-, XR-, Inhalt für einen Nutzer, wobei das Computerprogramm einen Computercode umfasst, der, wenn er auf einer Verarbeitungsschaltung (110) einer Vorrichtung (20) läuft, die Vorrichtung (20) veranlasst:
- den XR-Inhalt zu empfangen,
- den XR-Inhalt zu bestimmen, der basierend auf einer oder mehreren Richtlinien gerendert werden soll,
- den XR-Inhalt zu klassifizieren, und
- den XR-Inhalt in einer XR-Umgebung basierend auf einer oder mehreren Richtlinien und der Klassifizierung vom XR-Inhalt zu rendern,
wobei der zu rendernde XR-Inhalt mindestens basierend auf einem Schwellenwert für die Empfänglichkeit des Nutzers gegenüber XR-Inhalt im Hinblick auf einen aktuellen Kontext und auf eine Art einer Nutzeraufgabe, wobei der Wert der Empfänglichkeit des Nutzers durch Sensoren gemessen wird.

10. Vorrichtung (20) zum Bereitstellen von Extended-Reality-, XR-, Inhalt für einen Nutzer, wobei die Vorrichtung konfiguriert ist, um:
- XR-Inhalt zu empfangen,
- basierend auf einer oder mehreren Richtlinien zu bestimmen, welcher XR-Inhalt gerendert werden soll,
- den XR-Inhalt zu klassifizieren,
- XR-Inhalt in einer XR-Umgebung basierend auf einer oder mehreren Richtlinien und der Klassifizierung vom XR-Inhalt zu rendern,
wobei der zu rendernde XR-Inhalt mindestens basierend auf einem Schwellenwert für die Empfänglichkeit des Nutzers gegenüber XR-Inhalt im Hinblick auf einen aktuellen Kontext und auf eine Art einer Nutzeraufgabe, wobei der Wert der Empfänglichkeit des Nutzers durch Sensoren gemessen wird.

11. Vorrichtung (20) nach Anspruch 10, die konfiguriert ist, um den XR-Inhalt während eines XR-Ereignisses in der XR-Umgebung dynamisch zu bestimmen.

12. Vorrichtung (20) nach Anspruch 10 oder 11, die konfiguriert ist, um:
- in einem Überwachungsdienst (28) Daten in Bezug auf den Nutzer zu überwachen,
- im Überwachungsdienst (28) die Daten zu verarbeiten,
- die verarbeiteten Daten der Richtlinieneinheit (22) bereitzustellen, und
- die Richtlinieneinheit (22) basierend auf den verarbeiteten Daten zu aktualisieren.

13. Vorrichtung (20) nach Anspruch 12, die konfiguriert ist, um mindestens teilweise basierend auf vom Überwachungsdienst (28) bereitgestellten Daten zu bestimmen.

14. Vorrichtung (20) nach einem der Ansprüche 10 - 13, die konfiguriert ist, um basierend auf der aktuellen bestimmten kognitiven Belastung des Nutzers zu bestimmen, ob bestimmter XR-Inhalt gerendert werden soll.

15. Vorrichtung (20) nach einem der Ansprüche 10 - 14, die konfiguriert ist, um die aktuelle kognitive Belastung des Nutzers kontinuierlich oder intermittierend zu bestimmen, wobei die kognitive Belastung basierend auf einem oder mehr bestimmt wird von: Nutzerstandort, Nutzeraktivitätsniveau, biometrischen Daten, Bewegungsmuster eines Nutzers und Art der Nutzeraktivität.

16. Verfahren (200) zum Rendern von Extended-Reality-, XR-, Inhalt für einen Nutzer, wobei das Verfahren (200) in einem System (300) durchgeführt wird und umfasst:
- Empfangen (202) von XR-Inhalt,
- Bestimmen (204) des XR-Inhalts, der basierend auf einer oder mehreren Richtlinien gerendert werden soll,
- Klassifizieren (206) des XR-Inhalts, und
- Rendern (208) des XR-Inhalts in einer XR-Umgebung basierend auf einer oder mehreren Richtlinien und der Klassifizierung des XR-Inhalts,
wobei das Verfahren **dadurch gekennzeichnet ist, dass**:
das Bestimmen (12) mindestens auf einem Schwellenwert für die Empfänglichkeit des Nutzers gegenüber XR-Inhalt im Hinblick auf einen aktuellen Kontext und eine Art einer Nutzeraufgabe basiert, wobei der Wert der Empfänglichkeit des Nutzers durch Sensoren gemessen wird; und
wobei mindestens einer der Verfahrensschritte in einer Cloud-Rechenumgebung durchgeführt wird.

## Revendications

1. Procédé (10) de rendu de contenu de réalité étendue, XR, à un utilisateur, le procédé (10) étant mis en œuvre dans un dispositif (20), et comprenant :
- la réception (11), dans le dispositif (20), de contenu XR,
- la détermination (12), dans une entité de politique (22), du contenu XR à rendre sur la base d'une ou plusieurs politiques,
- la classification (13), dans un service de classification (24), du contenu XR, et
- le rendu (14) du contenu XR dans un environnement XR sur la base des une ou plusieurs politiques et de la classification du contenu XR, le procédé étant **caractérisé en ce que** :
la détermination (12) est basée au moins sur un seuil de sensibilité de l'utilisateur au contenu XR compte tenu du contexte actuel et du type de tâche de l'utilisateur, étant la valeur de sensibilité de l'utilisateur mesurée par des capteurs.

2. Procédé (10) selon la revendication 1, dans lequel la détermination (12) du contenu XR est effectuée dynamiquement au cours d'un événement XR dans l'environnement XR.

3. Procédé (10) selon la revendication 1 ou 2, comprenant
- la surveillance (15), dans un service de surveillance (28), de données relatives à l'utilisateur,
- le traitement (16), dans le service de surveillance (28), des données,
- la fourniture (17) des données traitées à l'entité de politique (22), et
- la mise à jour (18) de l'entité de politique (22) sur la base des données traitées.

4. Procédé (10) selon la revendication 3, dans lequel la détermination (12) est, au moins en partie, effectuée sur la base des données fournies par le service de surveillance (28).

5. Procédé (10) selon l'une quelconque des revendications précédentes, dans lequel le rendu (14) comprend la détermination de la nécessité de rendre un contenu XR particulier sur la base de la charge cognitive actuelle déterminée de l'utilisateur, dans lequel la charge cognitive actuelle de l'utilisateur est déterminée de manière continue ou intermittente, la charge cognitive étant déterminée sur la base d'un ou plusieurs parmi : l'emplacement de l'utilisateur, le niveau d'activité de l'utilisateur, les données biométriques, le modèle de mouvement de l'utilisateur et le type d'activité de l'utilisateur.

6. Procédé (10) selon l'une quelconque des revendications précédentes, dans lequel la détermination (12) est basée sur un ou plusieurs parmi : la fiabilité du contenu XR, l'utilisateur atteignant un seuil de charge cognitive d'utilisation, l'emplacement de l'utilisateur, le type d'activité de l'utilisateur, les préférences de l'utilisateur, l'historique des interactions de l'utilisateur avec d'autres utilisateurs, le contenu autorisé/bloqué, les métadonnées du contenu autorisé/bloqué, les ajouts cryptographiques au contenu autorisés/bloqués, la pertinence estimée pour l'utilisateur, le niveau d'activité de l'utilisateur, l'utilisateur immobile, au repos ou en mouvement, l'utilisateur dans un véhicule, l'utilisateur à l'extérieur ou à l'intérieur, l'heure de la journée.

7. Procédé (10) selon l'une quelconque des revendications précédentes, dans lequel la classification (13) comprend la détermination d'un ou plusieurs parmi : une catégorie à laquelle appartient le contenu XR, une valeur de confiance pour au moins une catégorie spécifique et une recommandation pour un contenu futur de même origine.

8. Procédé (10) selon l'une quelconque des revendications précédentes, dans lequel le service de classification (24) comprend un ou plusieurs parmi : un algorithme de détection d'images, un algorithme de détection de texte, un algorithme de détection d'objets, une base de données de contenu et de classification connexe respective, des jetons non fongibles avec des métadonnées stockées sur une chaîne de blocs, un algorithme de détection de la parole.

9. Programme informatique (100) de rendu de contenu de réalité étendue, XR, à un utilisateur, le programme informatique comprenant un code informatique qui, lorsqu'il est exécuté sur le circuit de traitement (110) d'un dispositif (20), amène le dispositif (20) à :
- recevoir le contenu XR,
- déterminer le contenu XR à rendre sur la base d'une ou plusieurs politiques,
- classer le contenu XR, et
- rendre le contenu XR dans un environnement XR sur la base des une ou plusieurs politiques et de la classification du contenu XR,
dans lequel le contenu XR à rendre est déterminé sur la base au moins d'un seuil de sensibilité de l'utilisateur au contenu XR compte tenu du contexte actuel et du type de tâche de l'utilisateur, étant la valeur de sensibilité de l'utilisateur mesurée par des capteurs.

10. Dispositif (20) destiné à fournir du contenu de réalité étendue, XR, à un utilisateur, le dispositif étant configuré pour :
- recevoir le contenu XR,
- déterminer le contenu XR à rendre sur la base d'une ou plusieurs politiques,
- classer le contenu XR,
- rendre le contenu XR dans un environnement XR sur la base des une ou plusieurs politiques et de la classification du contenu XR,
dans lequel le contenu XR à rendre est déterminé sur la base au moins d'un seuil de sensibilité de l'utilisateur au contenu XR compte tenu du contexte actuel et du type de tâche de l'utilisateur, étant la valeur de sensibilité de l'utilisateur mesurée par des capteurs.

11. Dispositif (20) selon la revendication 10, configuré pour déterminer dynamiquement le contenu XR au cours d'un événement XR dans l'environnement XR.

12. Dispositif (20) selon la revendication 10 ou 11, configuré pour :
- surveiller, dans un service de surveillance (28), des données relatives à l'utilisateur,
- traiter, dans le service de surveillance (28), les données,
- fournir les données traitées à l'entité de politique (22), et
- mettre à jour l'entité de politique (22) sur la base des données traitées.

13. Dispositif (20) selon la revendication 12, configuré pour déterminer, au moins en partie, sur la base des données fournies par le service de surveillance (28).

14. Dispositif (20) selon l'une quelconque des revendications 10-13, configuré pour déterminer s'il faut rendre un contenu XR particulier, sur la base de la charge cognitive actuelle déterminée de l'utilisateur.

15. Dispositif (20) selon l'une quelconque des revendications 10-14, configuré pour déterminer la charge cognitive actuelle de l'utilisateur de manière continue ou intermittente, dans lequel la charge cognitive est déterminée sur la base d'un ou plusieurs parmi : l'emplacement de l'utilisateur, le niveau d'activité de l'utilisateur, les données biométriques, le modèle de mouvement de l'utilisateur et le type d'activité de l'utilisateur.

16. Procédé (200) de rendu de contenu de réalité étendue, XR, à un utilisateur, le procédé (200) étant mis en œuvre dans un système (300) et comprenant :
- la réception (202) de contenu XR,
- la détermination (204) du contenu XR à rendre sur la base d'une ou plusieurs politiques,
- la classification (206) du contenu XR, et
- le rendu (208) du contenu XR dans un environnement XR sur la base des une ou plusieurs politiques et de la classification du contenu XR,
le procédé étant **caractérisé en ce que** :
la détermination (12) est basée au moins sur un seuil de sensibilité de l'utilisateur au contenu XR compte tenu du contexte actuel et du type de tâche de l'utilisateur, étant la valeur de sensibilité de l'utilisateur mesurée par des capteurs ; et
dans lequel au moins une des étapes du procédé est réalisée dans un environnement d'informatique en nuage.
